(19) Europäisches Patentamt / European Patent Office / Office européen des brevets

(11) **EP 3 745 477 A1**

(12) **EUROPEAN PATENT APPLICATION**

(43) Date of publication:
**02.12.2020 Bulletin 2020/49**

(51) Int Cl.:
***H01L 33/50*** (2010.01)      ***C07K 17/04*** (2006.01)

(21) Application number: **19382428.1**

(22) Date of filing: **29.05.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fundación Imdea Materiales
28906 Getafe (Madrid) (ES)**

(72) Inventors:
• **COSTA RIQUELME, Rubén Darío**
 **E-28320 Pinto, Madrid (ES)**
• **FERNÁNDEZ BLÁZQUEZ, Juan Pedro**
 **E-28905 Getafe, Madrid (ES)**
• **ESPASA VALDEPEÑAS, Anna**
 **E-28907 Getafe,  Madrid (ES)**

(74) Representative: **ABG Intellectual Property Law,
S.L.
Avenida de Burgos, 16D
Edificio Euromor
28036 Madrid (ES)**

(54) **LONG-LIVING BIO LIGHT-EMITTING DIODE**

(57)    The present invention refers to a rigid material composition containing a luminescent protein immobilized therein, a process for preparing it, as well as to a hybrid light emitting-diode comprising a light-emitting diode and a color down-converting encapsulation material based on a hydrophilic matrix, said color down-converting encapsulation material comprise at least one layer of said rigid material composition containing a luminescent protein immobilized therein.

## Description

## FIELD OF THE INVENTION

The present invention relates to the field of hybrid light-emitting diodes (also known as LEDs) and more particularly to LEDs based on materials containing luminescent proteins immobilized in a rigid hydrophilic polymeric matrix.

## BACKGROUND

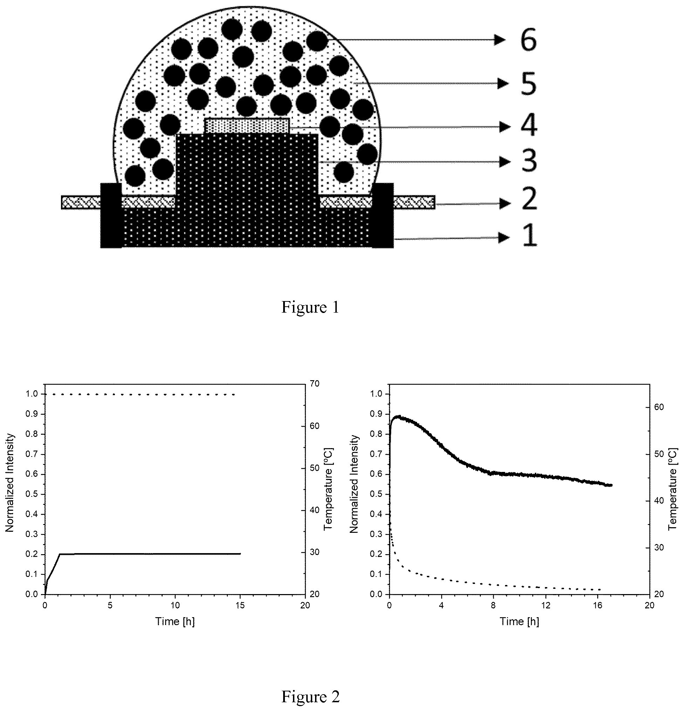

White light-emitting diodes (WLEDs) are considered a key solid-state lighting source to replace energy inefficient incandescent light bulbs and environmentally not friendly fluorescent lamps in the near future. They consist of inorganic-based color down-converting materials, most frequently phosphors of rare-earth elements (Park JK et al., Appl Phys Lett. 2004, 84, 1647; Jang HS et al., Appl Phys Lett. 2007, 90, 041906; Xie R-J et al., Nitride Phosphors and Solid-State Lighting, CRC Press, 2011; Tolhurst TM et al., Adv Opt Mater. 2015, 3, 546; Zhang R et al., Laser Photon Rev. 2014, 8, 158). As shown in Figure 1, WLEDs consist of four main components, namely i) the electronic driver, ii) the heat dissipating system, the emitting chip mounted onto the heat sink, iii) a packaging (encapsulation and protection layer) for environmental protection and optical purposes - *i.e.*, light extraction and color conversion, and iv) the color down-converting compound either placed direct onto the emitting chip or dispersed in the encapsulation part.

Although these devices show excellent device stabilities, there are several limitations:

> i) *Color quality*: The most efficient WLEDs relay on a blue-emitting GaInN-chip covered by a color down-converting inorganic phosphor based on yellowish orange-emitting rare-earth doped yttrium aluminum garnets, showing poor color quality with color rendering index (CRI) values *ca.* 75-80 - *i.e.*, bluish-white or cold white (J. Am. Ceram. Soc., 2014, 97, 1327-1352; J. Sol. State Light 2014 1, 11). This has both visual and non-visual effects on the human health like, for example, the disruption of the circadian rhythm (Environ. Health Perspect. 2014, 122, 269; Curr. Pharm. Des. 2015, 21, 3428; Neuroscience 2016, 339, 296; In Innovative Materials: Engineering and Applications II; Key Engineering Materials; Trans Tech Publications, 2017, 730, 112]. However, radiation intensities of *ca.* 100 kW/m$^2$ of blue-chips have blocked the use of organic color down-converting phosphors up to date.

> ii) *Costs and sustainability of LED components.* WLEDs still represent less than 0.1 % of the worldwide lighting waste stream (Lighting Research & Technology 2017 https://doi.org/10.1177/1477153517708597). In fact, they are based on rare-earth and/or nobel elements that are scarce and its production is associated to an ecological impact regarding mining, transporting, refining at high temperatures, *etc.* Two major concerns are the cost and the potentially limited supply. As such, the EU Sustainable Development Strategy Commission strongly encourages their further advance using eco-friendly and highly stable optical systems and active materials, as well as the establishment of efficient and low-cost recycling protocols for LEDs.

These limitations have led to a new research field called hybrid inorganic/organic LEDs, in which the inorganic phosphors are replaced for new organic-based color down-converting coating based on sustainable and non-toxic compounds, such as luminescent polymers, carbon quantum dots, carbon nanodots, small molecules, coordination complexes, and, more recently fluorescent proteins (Heliotis G et al., Adv Mater. 2006, 18, 334; Gu E. et al., Appl Phys Lett. 2007, 90, 031116; Huyal IO et al., J Mater Chem. 2008, 18, 3568; Kim O et al., ACS Nano 2010, 4, 3397; Stupca M et al., J Appl Phys. 2012, 112, 074313; Ban D et al., Phys Status Solidi Curr Top Solid State Phys. 2012, 9, 2594; Jang E-P et al., Nanotechnology 2013, 24, 045607; Findlay NJ et al., J Mater Chem C 2013, 1, 2249; Lai C-F et al., Opt Lett. 2013, 38, 4082; Chen J et al., J Mater Sci. 2014, 49, 7391; Kim J-Y, Opt Commun. 2014, 321, 86; Shen P-C et al., Sci Rep. 2014, 4, 5307; Findlay NJ et al., Adv Mater. 2014, 26, 7290).

Common to all these devices is the use of a similar LED architecture - *i.e.*, high-energy emitting inorganic LED with maxima emission wavelength ranging from 360 to 470 nm-, in which the current packaging system is replaced by a new coating based on a matrix and the organic color down-converting. Typical matrix components involve: i) thin films with a mixture of organic materials with UV- or thermal-curable sealing reagents (Adv. Mater., 2006, 18, 334; J. Mater. Chem., 2008, 18, 3568; ACS Nano, 2010, 4, 3397; Nanotechnology, 2013, 24, 045607; Sci. Rep., 2014, 4, 5307; Adv. Mater., 2014, 26, 7290; Nanoscale, 2015, 7, 12045). They are typically deposited either onto a glass substrate or on top of the packing of the LED. Other typical matrix includes metal-organic frameworks (Nat. Commun., 2013, 4, 2717; Adv. Funct. Mater., 2015, 25, 4796), cellulose/silk/mucin derivatives (J. Mater. Chem. C, 2015, 3, 3536; ACS Appl. Mater. Interfaces, 2015, 7, 15830.), and an elastomeric coating based on physical cross-linked polymers to embed all kind of luminescent compounds (Mater. Horiz., 2016, 3, 340) including fluorescent proteins (Adv. Mater., 2015, 27, 5493; Adv. Funct. Mater., 2017, 27, 1601792). This architecture has recently led to hybrid LEDs with high color quality - *i.e.*, commission international de l'Eclairage (CIE) coordinates of 0.30-3/0.30-3, color rendering index (CRI) above 90, and correlated color temperature (CCT) between 2500-6500 K, but still with low stabilities of around 100 h due to degradation of the luminescent material, the matrix, or both upon continuous excitation under ambient conditions.

Within the field of hybrid LEDs, particular mention should be made to those based on fluorescent proteins as color down-converting components, hereafter called Bio-LEDs.

The Bio-LED technology based on the use of said fluorescent proteins is restricted to a handful number of contributions which are herein below described.

Hui, K.N. (Nanotechnology, 2008, 19, 355203) discloses Bio-LEDs based on fluorescent proteins entrapped into polystyrene microspheres and deposited onto the LED emitting chip. A further protective layer - *i.e.*, epoxy - is deposited onto the thin protein-based thin film. As such, this procedure consists of two fabrication steps, being the polystyrene microspheres with a fluorescent protein core a commercial hybrid compound needed due to the incompatibility between said fluorescent proteins and the final protective epoxy layer. However, the matrix stability is not described and, in fact, the presence of an epoxy protective layer is required.

WO2017/039406 also describes a biomaterial comprising fluorescent proteins and a protective layer made of an epoxy resin. Since, as mentioned before, proteins are incompatible with an epoxy layer as they cannot endure in such a polymer resin, they are mixed with egg white leading to a hybrid protein-DNA$_{egg}$ in which the fluorescent proteins are cross-linked to the DNA present in the egg white. Furthermore, the addition of a surfactant is needed to produce a precipitate that is collected and further dispersed in the protective layer, although it is unclear the effect of said surfactant as stabilizer to allow the use of commercial epoxy. Anyway, there is no description of a polymer matrix to stabilize the fluorescent proteins (not only to protect them) or to a rigid coating lacking the use of standard protective layers of epoxy compounds. Furthermore, this type of Bio-LEDs does not circumvent the problems mentioned above. In addition to that, the device stability, efficiency and color quality are not disclosed.

Bio-LEDs based on a dried film of fluorescent proteins without using any matrix component and/or a further encapsulation is described in Fen Bilim. Enstitüsü Derg., 2016, 20, 490; and Nanotechnology 2016, 27, 45LT01. The device stability is not disclosed, but the authors only commented on the possibility to enhance the device stability using additives like polysaccharides.

WO2016/203028 (see also Adv. Mater., 2015, 27, 5493; Adv. Funct. Mater., 2017, 27, 1601792; ACS Omega 2018, 3, 15829) describes Bio-LEDs based on fluorescent proteins (monomers and trimers) entrapped into a rubber-like coating based on a mixture of a branched and a linear polyethylene oxide compounds in aqueous solution. This coating replaces the commercial packaging and it does not include a further encapsulation. The procedure and examples disclosed relates to the use of hydrophilic branched and linear polymers that are mixed in aqueous solutions containing the fluorescent proteins. Upon drying under vacuum conditions, the gel-like materials transform into a rubber-like and/or elastomeric material.

Although Bio-LEDs based on a rubber-like and/or elastomeric fluorescent protein packaging show less than 10% loss in luminous intensity over a 100 h upon driving the device at low applied currents (10 mA), they show a low stability of a few minutes (< 5 min) upon driving the device at high applied currents (200 mA). A rationale, which has not been disclosed in the literature yet, is the high temperature reached by the coating upon continuous excitation.

The increase in temperature is detrimental with respect to the intrinsically low thermal stability of fluorescent proteins and the rapid loss of water molecules embedded into the elastomeric matrix. Both aspects are limitations of the rubber-like material in terms of low thermal conductivity and high degree of flexibility that facilitates the loss of water. An obvious solution is the use of a protective layer covering the rubber-coating as described in other examples (Nanotechnology, 2008, 19, 355203). However, this is not desired as it increases the number of fabrication steps and it could potentially be incompatible due to the use of hydrophobic compounds for both matrix and protective layers, being fluorescent proteins only stable enough for lighting applications using aqueous solutions or elastomeric materials containing a high percentage of water molecules.

Another technique might involve the change of the device architecture placing the coating at certain distance from the emitting inorganic chip (remote architecture). This option is also not desirable since the device efficiency is strongly reduced due to the low transmittance values of the elastomeric coating.

As such, the main technical challenges in the field of Bio-LEDs based on fluorescent proteins as color down-converting packaging are:

- need of a single-layer encapsulation without using a further protective layer;
- need of a simple fabrication procedure not limited to the use of two-component polymers to simultaneously stabilize the fluorescent proteins and to provide excellent mechanical and optical features for LED packaging;
- need of a new encapsulation concept to reduce the thermally induced loss of water to preserve the Bio-LED stability under high applied currents.

None of the disclosed documents have proposed methods to solve the issues above. As such, there is a strong need of Bio-LEDs having higher stabilities, avoiding the degradation of the fluorescent proteins upon continuous excitation under ambient conditions and at high applied currents, as well as the use of simple packaging composition.

On the other hand, the use of poly(vinyl alcohol) (PVA) as packaging system is widely known in the state of the art. In general, the literature describes PVA composite films with different active dopants for industrial applications mainly for improving hardness, barrier properties, antibacterial or antioxidant properties without disclosing the reduction of the heat generation under external excitation stimulus like electricity, light, pressure, etc. (Int. J. Biol. Macromol., 2017, 104, 43; J. Appl. Polym. Sci., 2017, 134, 45324; Int. J. Biol. Macromol., 2017, 96, 340; Carbohydr. Polym., 2015, 127, 64).

PVA has also been used as a carrier media for biological materials, such as fluorescent proteins, DNA, cells, *etc.* The combination of fluorescent proteins-PVA composite films have been used for imaging purposes, in which the fluorescent proteins are well-mixed with the polymer to perform spectroscopic analysis by circular dichroism, single molecular spectroscopy, steady state spectroscopy techniques (Int. J. Polym. Mater., 2010, 56:10, 786; PNAS, 2005, 102(27), 9511; Proc SPIE Int. Soc. Opt. Eng., 2015, 9554, 95540G-1; Chem. Phys. Lett., 2008, 457, 408). The use of PVA hydrogels using cross-linking approach to immobilize fluorescent proteins, cells, DNA, *etc.* in a water ambient is also used for imaging and DNA/drug delivery purposes, although in this case a water media is needed to preserve hydrogel characteristics *(*Proc. SPIE, 2006, 6098; Otolaryngol. Head Neck Surg., 2016, 154(6), 1106; Biofouling, 2000, 15(1-3), 109; Curr. Protoc. Cell Biol., 2009, 10, 15; Langmuir, 2015, 21(23), 10253; J. Biomater. Sci. Polym. Ed., 2012, 15(9), 1181). None of these documents describe the use of fluorescent proteins and polyvinyl alcohol derivatives for color down-converting purposes and even less its use as packaging systems in light-emitting diodes. In addition, the effect of the polymeric matrix on the changes of the temperature upon spectroscopic inspection has not been described. This might be related to the high level of water surrounding the biomaterials, in general, and the fluorescent proteins, in particular. As well, the use of PVA derivatives either to reduce temperature stress or to avoid temperature increase has not been noted.

The use of PVA has been proposed as LED packaging (Front. Optoelectron., 2012, 5(2), 147; Chem. Phys. B, 2010, 19(11), 118103). Said PVA is crosslinked using ammonium dichromate. Here, the authors' motivation is the better thermal conductivity of the PVA with respect to silicone and/or epoxy. However, none of these documents discloses the use of PVA as a single component without cross-linking reactions, letting alone the incorporation of fluorescent proteins. In fact, the chemical cross-linking of the polymer would restrict the use of fluorescent proteins as described in the prior art of Bio-LEDs (Adv. Mater., 2015, 27, 5493; Adv. Funct. Mater., 2017, 27, 1601792; Nanotechnology, 2016, 27, 45LT01; Nanotechnology, 2008, 19, 355203; WO2016/203028).

Thus, the use of a rigid color down-converting packaging based on poly(vinyl alcohol) or derivatives thereof to stabilize fluorescent proteins has not been reported in the prior art.

## BRIEF DESCRIPTION OF THE INVENTION

The authors of the present invention have found a technical solution that simultaneously solves the above-discussed problems of the Bio-LEDs described in the prior art. More particularly, they have developed a rigid single-layer and single-component hydrophilic polymer coating in which the embedded luminescent proteins are stable over time, while enhancing optical and mechanical features meeting the requirement for LED packaging systems. In fact, the rigid polymer coating keeps the temperature of the coating below the critical denaturation temperature of the fluorescent proteins (<50°C) and, in turn, the loss of water of the coating at high applied currents. The hydrophilic nature of the polymer is key to further stabilize the luminescent proteins with respect to its intrinsic photo-induced degradation. The combination of both aspects has surprisingly led to a 100-fold enhancement of the device stability compared to the current state of the art devices as pointed out in the comparative example herewith provided.

Furthermore, the Bio-LEDs can be prepared by a very simple procedure based on the combination of fluorescent proteins with a rigid hydrophilic polymer using water or a hydroalcoholic solution as the sole solvent, thus without affecting the stability of said proteins caused by the use of hydrophobic polymers (e.g., polystyrene, PMMA, etc.) and non-polar (e.g., toluene, chlorobenzene, etc.) and/or aprotic polar (e.g., acetonitrile, dichloromethane, etc.) solvents as in previous inventions.

Thus, a first aspect of the present invention refers to a hybrid light emitting-diode comprising a light-emitting diode and a rigid color down-converting encapsulation material, said rigid color down-converting encapsulation material comprises at least one layer of a rigid material composition, wherein said rigid material composition comprises at least one hydrophilic thermoplastic polymer having a glass transition temperature higher than 30°C and a luminescent protein immobilized therein.

Single components are preferable for practical uses, but multicomponent hydrophilic thermoplastic polymer are equally operative as shown in the examples herein provided. Thus, in a preferred embodiment, the rigid material composition comprises a single (or one) hydrophilic thermoplastic polymer having a glass transition temperature higher than 30°C.

In other particular embodiment, the rigid material composition comprises more than one hydrophilic thermoplastic polymer having a glass transition temperature higher than 30°C.

The use of a rigid color down-converting encapsulation material based on a hydrophilic polymer to stabilize fluorescent proteins effectively eliminates the issues associated to the state-of-the-art rubber-like fluorescent protein based color down-converting coatings as it allows:

- the suppression of the heat generation under device operation conditions;

- the reduction of the overall temperature of the coating under device operation conditions;

- the reduction of the loss of water content over time;

- the elimination of the protective layer due to the poor mechanical properties of the rubber coating and the ease of water loss;

- the great enhancement in device stability under high applied currents.

These improvements of the device performance are achieved while also providing improvements in the coating fabrication:

- possibility of using a simple coating formulation with one polymer;

- possibility of using water based polymer solutions that are completely dried after a vacuum cycle;

- possibility of using a single polymer with fluorescent proteins beyond the prior art limited to the use of a mixture of polymers that retain water in its matrix;

- simple device fabrication since there is no need of a protective layer.

In a preferred embodiment, the rigid hydrophilic thermoplastic polymer having a glass transition temperature higher than 30°C is poly(vinyl alcohol), a derivative thereof or its copolymers.

In a second aspect the invention refers to a process for preparing the hybrid light emitting-diode, said process comprises:

a) preparing an aqueous solution or a hydroalcoholic solution of a hydrophilic polymer having a glass transition temperature higher than 30°C;

b) preparing an aqueous solution of a luminescent protein;

c) mixing the solutions prepared in steps a) and b) to obtain a hydrogel;

d) depositing the hydrogel obtained in step c) onto a light emitting diode;

e) drying the hydrogel under vacuum conditions to obtain a rigid material composition containing the luminescent protein immobilized therein.

A third aspect of the invention relates to a hybrid light emitting-diode obtainable by the process as defined above.

Another aspect of the relates to the use of a rigid material composition as an environmentally friendly color down-converting encapsulation material for a hybrid light-emitting diode, wherein said rigid material composition comprises a rigid hydrophilic thermoplastic polymer having a glass transition temperature higher than 30°C and a luminescent protein immobilized therein. The luminescent protein contained in the rigid material composition is preferably a fluorescent protein.

**BRIED DESCRIPTION OF THE FIGURES**

Figure 1 shows a sketch of a Bio-hybrid light-emitting diode (Bio-LED) according to the invention.

Figure 2. (a) Device performance of a LED with a rubber-like coating without protein, showing the emission intensity of the LED (dotted line) and the temperature of the coating (solid line) upon driving currents of 200 mA. (b) Device performance of a Bio-LED with a rubber-like packaging, showing the normalized decay of the emission intensity (dotted line) of the fluorescent protein and the rise of the temperature (solid line) of the coating upon driving at a current of 200 mA.

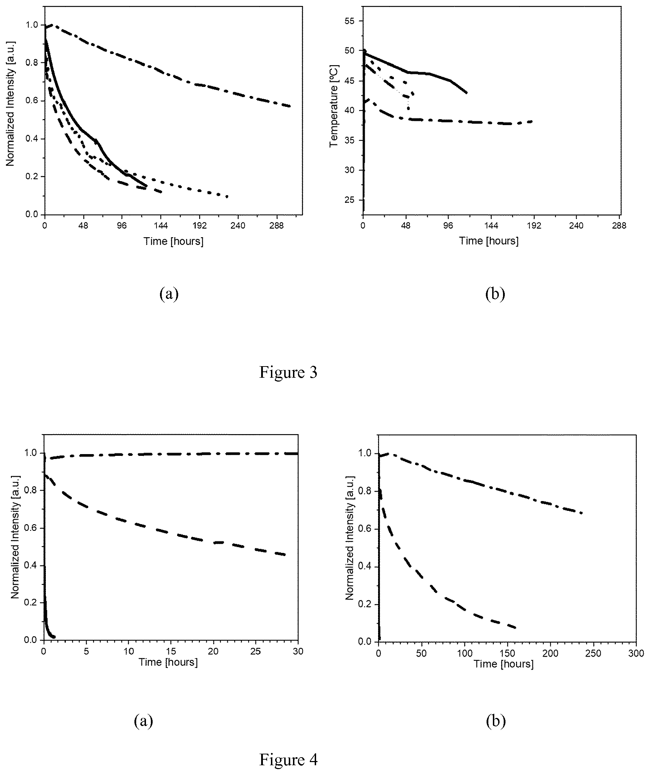

Figure 3 shows the device performance of a Bio-LED according to the present invention with a rigid coating based on different PVAs with different hydroxylation degree and molecular weights: Mowiol 10-98 (dashed line), Mowiol 28-99 (dotted line), Mowiol 56-98 (solid line), and Mowiol 18-88 (dash-dotted line) showing the normalized decay of the emission intensity (a) of the fluorescent protein and the rise of the temperature (b) of the coating upon driving at a current of 200 mA.

Figure 4 shows the device performance of a Bio-LED with: a elastomeric packaging (solid line); and a rigid packaging based on a one component matrix according to the invention operating at temperature above 40 °C (dashed line) and below 40 °C (dashed-dot line), showing the normalized decay of the emission intensity of the fluorescent protein upon driving at currents of 200 mA at short (a) and large (b) measuring times.

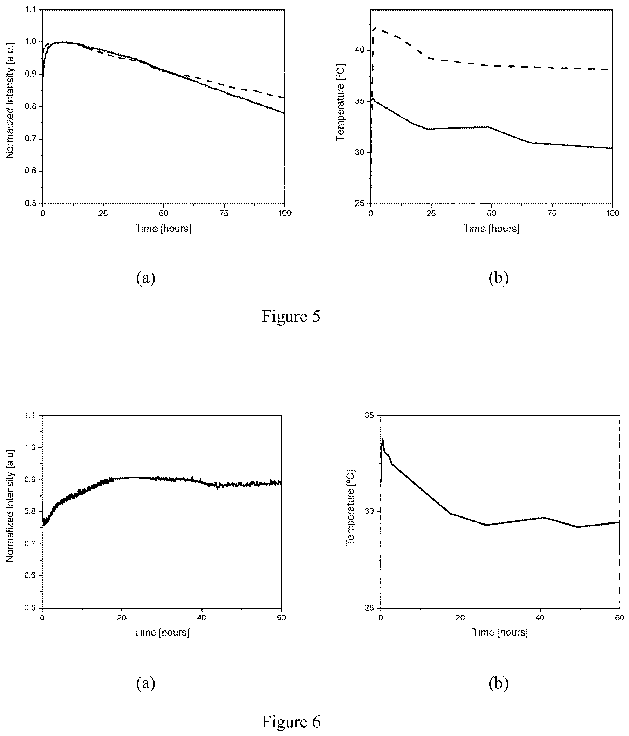

Figure 5 shows the device performance of a Bio-LED with a rigid packaging based on a one component matrix prepared with Mowiol 18-88 in water (dashed lines) and in a mixture of water/alcohol (1:1 v/v) (solid lines), showing the normalized decay of the emission intensity (a) of the fluorescent protein and the rise of the temperature (b) of the coating upon driving at a current of 200 mA.

Figure 6 shows the device performance of a Bio-LED with a rigid packaging based on a one component matrix prepared with PVA co-polymer in a mixture of water/alcohol (1:1 v/v), showing the normalized decay of the emission intensity (a) of the fluorescent protein and the rise of the temperature (b) of the coating upon driving at a current of 200 mA.

## DETAILED DESCRIPTION OF THE INVENTION

As mentioned above, the first aspect of the invention relates to a hybrid light emitting-diode (hybrid LED) comprising a light emitting diode (LED) and a rigid color down-converting encapsulation material, said rigid color down-converting encapsulation material comprises at least one layer of a rigid material composition, wherein said rigid material composition comprises at least one hydrophilic thermoplastic polymer having a glass transition temperature higher than 30°C and a luminescent protein immobilized therein.

The hybrid light-emitting diode of the invention will also be referred as to Bio-LED in the present document due to the presence of a protein immobilized therein.

In the context of the present invention, the term "color down-converting encapsulation material" refers to an encapsulation material that absorbs incident radiation having a relatively shorter wavelength and re-emits radiation at a relatively longer wavelength.

The term "rigid material composition" refers to a material composition that is essentially non-flexile under conventional forces exerted by a person.

In a particular embodiment, the invention refers to a hybrid light emitting-diode comprising a light emitting diode and a rigid color down-converting encapsulation material, said rigid color down-converting encapsulation material comprises at least one layer of a rigid material composition, wherein said rigid material composition comprises a hydrophilic thermoplastic polymer having a glass transition temperature higher than 30°C and a luminescent protein immobilized therein.

In another particular embodiment, the invention refers to a hybrid light emitting-diode comprising a light emitting diode and a rigid color down-converting encapsulation material, said rigid color down-converting encapsulation material comprises at least one layer of a rigid material composition, wherein said rigid material composition consists of a hydrophilic thermoplastic polymer having a glass transition temperature higher than 30°C and a luminescent protein immobilized therein.

In another particular embodiment of the invention, the rigid down-converting encapsulation material comprises or consists of several layers (e.g., two, three, four, five, six, seven, eight, or more layers) of the rigid material containing the luminescent protein immobilized therein, wherein the luminescent proteins in each pair of neighbouring layers have complementary absorption and emission features.

In another particular embodiment, the hybrid light-emitting diode comprises a bottom light emitting inorganic chip or diode (LED), optionally a first encapsulation on the inorganic LED, and a second encapsulation composed of the rigid material composition comprising the hydrophilic thermoplastic polymer having a glass transition temperature higher than 30°C and a luminescent protein immobilized therein. This second encapsulation is preferably multi-layered, and may be placed directly on top of the inorganic LED or on top of the first encapsulation (which may be made of an organic/or an inorganic material, and may have any 3D form). Upon excitation by the inorganic LED, such luminescent proteins partially convert the high-energy photons into low-energy photons, which sum up to the non-absorbed high-energy photons from the inorganic LED, resulting in a color change of the inorganic LED.

Figure 1 shows a hybrid light-emitting diode according to an embodiment of the present invention, which comprises a substrate (1) with electrical connections, in particular a leadframe (2) to be connected to an electronic driver, a heat sink (3), a light-emitting inorganic chip (4) which can be blue- or UV-emitting chip, and a down-converting encapsulation system (5) that consists of one or several layers of the rigid material composition containing the luminescent protein (6) immobilized therein.

The rigid material composition present in the hybrid light-emitting diode of the invention comprises a hydrophilic thermoplastic polymer having a glass transition temperature higher than 30°C and a luminescent protein immobilized therein.

The presence of the hydrophilic thermoplastic polymer having a Tg higher than 30°C provides a rigid material composition wherein the luminescent protein is immobilized. Said rigidity has found to impair a high thermal stability to the Bio-LED where this rigid material composition is incorporated as an encapsulation, as no temperature increase is observed, thus reducing significantly the degradability of luminescent proteins.

Furthermore, the loss of water is also remarkably reduced due to the rigidity of the resulting polymeric matrix. But even in the absence of water (as the rigid material can be completely dry, for example under vacuum conditions), the rigid-like encapsulation material gives the luminescent protein a good media to maintain its photoluminescent characteristics.

In fact, and as shown in the experimental results, a 100-fold enhancement (from a few minutes to more than 40 h) in the device stability has surprisingly been found in comparison to Bio-LEDs having a rubber-like encapsulation material.

Thus, the main advantages conferred by the use of a rigid color down-converting encapsulation material based on a single-component hydrophilic polymeric in comparison to devices using rubber-like color down-converting encapsulation materials relies on:

- the reduction of the temperature of the encapsulation material under device working conditions;

- the reduction of the loss of water content under device working conditions;

- the possibility of eliminating a protective layer due to the excellent mechanical properties of the rigid material and the lack of water content;

- the increased device stability under high applied currents.

As mentioned above, the hydrophilic thermoplastic polymer comprised in the material composition of the invention is characterized for having a Tg higher than 30°C, preferably higher than 40°C, more preferably higher than 50°C, even more preferably higher than 70°C. In other words, this thermoplastic polymer is one which at ambient temperature is solid and rigid. This component of the material composition is the responsible for the hardness and rigidity characteristics thereof.

In the context of the present invention, the values of "glass transition temperature" (Tg) refer to those taken from the second heating in differential scanning calorimeter (DSC) experiments. Said experiments can be carried out under non-isothermal (10°C/min from -85 to 200°C) conditions using a DSC TA Instruments Q200, operating with an intra-cooler under nitrogen flow. Temperature and heat flow calibrations are performed with indium as standard.

Also in the context of the invention, the "number average molecular weight" (or "Mn") of a polymeric component refers to the average (arithmetic mean) of the molecular weights of the individual molecules of the corresponding component (e.g., of the branched polymer or of the linear polymer). The number average molecular weight (Mn) is defined as follows:

$$Mn = \frac{\sum_i NiMi}{\sum_i Ni}$$

wherein Ni is the number of molecules of the respective component having a molecular weight Mi, and the summation includes all molecular weights of the corresponding component that are present. The number average molecular weight of a component (such as the branched polymer) can be determined using methods known in the art, such as e.g., by gel permeation chromatography (GPC), viscosity measurements (viscometry), osmotic-pressure measurements, light-scattering measurements (e.g., using the Zimm method), colligative methods (such as vapor-pressure osmometry, boiling-point elevation, freezing-point depression, or vapor-pressure lowering), end-group determination, or $^1$H-NMR.

It is preferred that the number average molecular weight is to be determined using GPC or viscosity measurements, more preferably by GPC. The GPC system can be calibrated, e.g., relative to a set of anionically polymerized polystyrenes having a dispersity Mw/Mn < 1.10 (ideally Mw/Mn = 1) as calibration standard. For example, the number average molecular weight can be determined by GPC, using a GPC apparatus C0-8011 (Tosoh Bioscience LLC) equipped with a column GMHHR-H (Tosoh Bioscience LLC), using tetrahydrofuran as the solvent, measuring at 40°C, and using polystyrenes as standard (e.g., as described above).

Water can also be used as the solvent instead of tetrahydrofuran in this method. Alternatively, the number average molecular weight can be determined by GPC, using a GPC apparatus 150C (Waters Corporation) equipped with a Shodex Packed Column A-80M (Showa Denko K.K.), measuring at 140°C, using ortho-dichlorobenzene as solvent/carrier, a flow rate of 1.0 mL/min, a sample concentration of about 1 mg/mL, an injection amount of 400 ml, a differential refraction detector, and polystyrenes as standard (e.g., as described above). It is particularly preferred that the number average molecular weight is determined by GPC, using a GPC apparatus 150C (Waters Corporation) equipped with a Shodex Packed Column A-80M (Showa Denko K.K.), measuring at 40°C, using water as solvent, a flow rate of 1.0 mL/min, a sample concentration of about 1 mg/mL, an injection amount of 400 ml, a differential refraction detector, and polystyrenes as standard (e.g., anionically polymerized polystyrenes having a dispersity Mw/Mn < 1.10).

The hydrophilic thermoplastic polymer has preferably a transparency degree of at least 90% in the region of the visible spectra in order to ensure a suitable transparency.

In another preferred embodiment, the hydrophilic thermoplastic polymer has a refractive index equal to or higher than 1.

In a particular embodiment of the invention, said hydrophilic thermoplastic polymer is selected from poly(vinyl alcohol), derivatives thereof and its copolymers, provided that they have a Tg higher than 30°C and a degree of transparency in the visible spectra higher than 90%.

Examples of derivatives of poly(vinyl alcohol) are polyvinyl acetals, such as polyvinyl butyral (PVB) and polyvinyl formal (PVF). They are prepared by reacting aldehydes with poly(vinyl alcohol). PVB and PVF are prepared from poly(vinyl alcohol) by reaction with butyraldehyde and formaldehyde, respectively. Poly(vinyl acetate) is also an example of poly(vinyl alcohol) derivative.

Examples of copolymers of these hydrophilic thermoplastic polymers are poly(vinyl alcohol-co-ethylene), poly(vinyl alcohol-*co*-vinyl acetate), poly(ethylene-*co*-vinyl acetate), poly(vinyl butyral-*co*-vinyl alcohol-*co*-vinyl acetate) or poly(vinyl chloride-*co*-vinyl acetate-*co*-vinyl alcohol).

Said hydrophilic thermoplastic polymers suitable of use in the invention are commercially available or can be prepared according to standard procedures known in the prior art.

Homopolymers of poly(vinyl alcohol) are preferred, although copolymers as those mentioned above are also possible.

Thus, in a preferred embodiment, the hydrophilic thermoplastic polymer is poly(vinyl alcohol). This polymer has repeating units of vinyl alcohol: $[CH_2CH(OH)]_n$. It can be prepared by first polymerizing vinyl acetate and the resulting polyvinyl acetate is converted to poly(vinyl alcohol) by base-catalysed transesterification with ethanol.

Poly(vinyl alcohol) is mainly composed of 1,3-diol linkages [-CH$_2$-CH(OH)-CH$_2$-CH(OH)-] but a few percent of 1,2-diols [-CH$_2$-CH(OH)-CH(OH)-CH$_2$-] occur, depending on the conditions for the polymerization of the vinyl ester precursor. In a preferred embodiment, the polyvinyl alcohol has a deacetylation degree higher than 80%, more preferably higher than 85%, even more preferably between 85% and 95%, most preferably between 85 and 90%. By deacetylation degree can be understood the acetate groups that are converted to hydroxyl groups when obtaining the polyvinyl alcohol from deacetylation of poly(vinyl acetate).

In a particular embodiment, the hydrophilic thermoplastic polymer is a copolymer comprising structural units of vinyl alcohol and at least one derivative monomer thereof, such as vinyl acetate, vinyl butyral, vinyl formal and vinyl chloride.

In another particular embodiment, the hydrophilic thermoplastic polymer is a copolymer comprising structural units of vinyl alcohol and ethylene, i.e., a poly(vinyl alcohol-co-ethylene). More preferably, the poly(vinyl alcohol-co-ethylene) comprises less than 30 wt% of ethylene units.

In another particular embodiment, the number average molecular weight (Mn) of the hydrophilic thermoplastic polymer ranges from $10^4$ to $10^7$ Da, more preferably from $10^4$ to $10^6$ Da, even more preferably from $10^4$ to $10^5$ Da. The number average molecular weight of the hydrophilic thermoplastic polymer should be such that the resulting material composition is rigid at ambient temperature.

In a preferred embodiment of the invention, the hydrophilic thermoplastic polymer is poly(vinyl alcohol) having a Mn ranging from 10.000 to 200.000 Da.

Luminescent protein

The protein to be used in accordance with the invention is a luminescent protein, more preferably a fluorescent protein.

The term "protein" is used herein interchangeably with "polypeptide" or "peptide" and refers to a polymer of two or more amino acids (preferably 10 or more amino acids, more preferably 20 or more amino acids, even more preferably 50 or more amino acids, even more preferably 100 or more amino acids, even more preferably 150 or more amino acids, and yet even more preferably 200 or more amino acids) linked via amide bonds that are formed between an amino group of one amino acid and a carboxyl group of another amino acid. In the context of the present invention, the protein is luminescent, i.e. spontaneously emits light upon radiative excitation.

The luminescent protein is preferably a fluorescent protein, such as, e.g., green fluorescent protein (GFP), enhanced green fluorescent protein, blue fluorescent protein, cyan fluorescent protein, teal fluorescent protein, yellow fluorescent protein, orange fluorescent protein, red fluorescent protein, near-infrared fluorescent protein, mCherry, mStrawberry, mRaspberry, mOrange, mCitrine, tdTomato, mTagBFP, dsRed, UnaG, eqFP611, Dronpa, TagRFPs, KFP, EosFP, Dendra, or IrisFP.

The fluorescent protein may also be a fusion protein comprising a fluorescent protein (e.g., any of the above-mentioned specific luminescent proteins), which is fused, optionally via a linker (e.g., a glycine and/or serine rich linker, such as a linker composed of 2 to 35 amino acids, preferably 5 to 10 amino acids, selected independently from glycine and serine, or any one of the linkers mentioned in Reddy Chichili VP et al., Protein Scí. 2013, 22(2):153-67, including in Table 1 of this reference), to an adaptor protein domain (e.g., an Src homology 2 domain (SH2 domain), an Src homology 3 domain (SH3 domain), or a poly(A)-binding protein C-terminal domain (PABC domain)). It is possible to use a single protein, i.e. a single type of protein, or to use two or more different proteins, e.g., two or more different luminescent proteins.

In a particular embodiment, the luminescence protein is a conventional protein that has been modified by covalent attachment of one or more non-protein fluorescent dyes (such as fluorescein, rhodamine, Oregon green, eosin, or Texas red) and/or one or more phosphorescent dyes. In this particular case, the protein comprises amino acids, which are also referred to as amino acid residues, that may be selected from the 20 standard proteinogenic α-amino acids (i.e., Ala, Arg, Asn, Asp, Cys, Glu, Gln, Gly, 25 His, Ile, Leu, Lys, Met, Phe, Pro, Ser, Thr, Trp, Tyr, and Val) but also from non-proteinogenic and/or non-standard α-amino acids (such as, e.g., ornithine, citrulline, homolysine, pyrrolysine, or 4-hydroxyproline) as well as β-amino acids (e.g., 13-alanine), γ-amino acids and δ-aminoacids. Preferably, the amino acid residues comprised in the protein are selected from α-aminoacids, more preferably from the 20 standard proteinogenic α-amino acids (which can be present as the L-isomer or the D-isomer, and are preferably all present as the L-isomer).

It is generally preferred that the luminescent protein is unmodified, unless explicitly indicated otherwise. The amino acid residues comprised in the luminescent protein may, e.g., be present as a linear molecular chain (forming a linear protein) or may form one or more rings (corresponding to a cyclic protein). The protein may also form oligomers consisting of two or more identical or different molecules. The protein may, e.g., comprise or consist of about 200 to about 800 amino acid residues, or it may have a molecular weight of about 20 kDa to about 800 kDa.

The invention also refers to a process for preparing the hybrid light emitting-diode as described above, said process comprises:

a) preparing an aqueous solution or a hydroalcoholic solution of a hydrophilic polymer having a glass transition temperature higher than 30°C;

b) preparing an aqueous solution of a luminescent protein;

c) mixing the solutions prepared in steps a) and b) to obtain a hydrogel;

d) depositing the hydrogel obtained in step c) onto a light emitting diode;

e) drying the hydrogel to obtain a rigid material composition containing the luminescent protein immobilized therein.

The aqueous solution to be used in steps a) and b) of the process of the invention is not particularly limited, and is preferably water or an aqueous buffer solution. Examples of suitable aqueous buffer solutions include, in particular, phosphate buffer, HEPES buffer, Tris buffer, MOPS buffer, MES buffer, TES buffer, CHES buffer, PIPES buffer, CAPS buffer, HEPPS buffer, imidazole buffer, tricine buffer, bicine buffer, glycine buffer, citric acid buffer, or acetic acid buffer. The pH of the buffer can be adjusted using, e.g., HCl or NaOH (or KOH) as desired for the protein to be immobilized, particularly to a pH at which the protein remains correctly folded (e.g., about pH 6, about pH 7, or about pH 8). A preferred exemplary aqueous buffer solution is phosphate-buffered saline (PBS), which can be prepared, e.g., according to the Cold Spring Harbar Protocol (doi:1 0.11 01/pdb.rec8247). It is particularly preferred that the protein is provided in an aqueous buffer solution containing 50 mM $NaH_2PO_4$ pH 8.0, 300 mM NaCl, and 250 mM imidazole (e.g., at a protein concentration of about 1 mg/ml to about 20 mg/ml). The aqueous buffer solution may further contain one or more protein stabilizers, such as poly(ethyleneimine) (PEI), ethylenediaminetetraacetic acid (EDTA), ammonium sulfate, trehalose, or a commercially available protein stabilizer such as "Thermo Scientific Protein Stabilizing Cocktail" (Life Technologies, product no. 89806). Any other aqueous solvent or aqueous medium (e.g., containing at least about 60 vol-% water, preferably at least about 70 vol-% water, more preferably at least about 80 vol-% water, even more preferably at least about 90 vol-% water, still more preferably at least about 95 vol-% water) can also be used in place of the above-described aqueous solution in order to ensure the stability of the protein.

Alternatively, a hydroalcoholic solution can also be prepared in step a) by using a mixture of water and an aliphatic alcohol, such as ethanol or propanol. The hydroalcoholic solution is more preferable when the hydrophilic polymer is a copolymer. The alcohol in the mixture should not be present in a percentage higher than 50% v/v.

In a preferred embodiment, step a) is conducted by mixing the hydrophilic thermoplastic polymer with the proper amount of the aqueous solution of the hydroalcoholic solution, preferably under stirring (e.g. 1500 rpm). It is furthermore preferred that step a) is conducted under high temperature, particularly, from about 35 to 50°C.

Step b) of the process of the invention can be conducted by dissolving the luminescent protein in the aqueous solvent, such as those mentioned above, under room conditions.

Step c) of the process of the invention can be conducted by simply mixing the solutions obtained in steps a) and b) described above under stirring and room conditions, thus leading to a protein-based hydrogel.

The solution prepared in step a) can be added to the aqueous solution obtained in step b) or viceversa.

Alternatively, both the hydrophilic thermoplastic polymer and the luminescent protein can be dissolved in the same solution, thus eliminating step c) of the process of the invention but also providing a protein-based hydrogel.

The use of an aqueous solvent, both in step a) and step b), is particularly advantageous as it ensures maintaining the stability of the luminescent protein without affecting the viability thereof. However, the use of a hydroalcoholic solution having less than 50 %v. of alcohol also allows the viability of said proteins.

In step d) of the process of the invention, the hydrogel formed in step c) is deposited onto a light-emitting chip or diode (LED) using for example any coating or printing method, particularly a solvent-based technique. Exemplary techniques for depositing the gel onto the LED include, in particular, doctor-blading, roll-to-roll coating, spin coating, gravure printing, inkjet printing, flexographic printing, screen printing, or 3D printing.

Step e) of the process of the invention comprises drying the hydrogel obtained in step c) and deposited onto the LED according to step d) in order to obtain a rigid material composition containing the luminescent protein immobilized therein which encapsulates the light emitting diode.

Preferably, the hydrogel is partially dehydrated via vacuum drying, freeze-drying, drum-drying, spray drying, or sunlight-ambient evaporation. It is particularly preferred that the hydrogel is partially dehydrated via vacuum drying.

Accordingly, it is preferred that in step e) the hydrogel is partially dehydrated in a vacuum station/chamber (e.g., at a pressure of about 1 mbar to about 10 mbar for a period of less than or equal to about 1 hour, or alternatively at a pressure of about $10^{-5}$ bar to about

10$^{-9}$ bar for a period of about 5 seconds to about 5 minutes).

In the process of the invention, the use of a hydrophilic thermoplastic polymer having a Tg higher than 30°C and the subsequent drying of the hydrogel formed has led to a rigid matrix which has shown to reduce the temperature of the Bio-LED where it is incorporated under working conditions, thus reducing the loss of water and degradability of proteins.

Furthermore, the process according to the present invention allows the preparation of a material composition containing a protein immobilized therein without the need for any crosslinking or curing of the polymer that is used. It is thus preferred that the process of the invention does not comprise any step of thermally curing, UV curing or crosslinking the hydrophilic thermoplastic polymer that constitutes the material composition. It is likewise preferred that the process does not comprise any step of covalently crosslinking the hydrophilic thermoplastic polymer that constitutes the material composition.

The rigid material composition obtained by drying the hydrogel prepared in step c) and deposited onto the LED according to step d) can be prepared in the form of a film having a thickness of about 10 nm to about 20 mm, preferably of about 10 $\mu$m to 10 mm.

The invention also relates to the use of a rigid material composition as an environmentally friendly color down-converting encapsulation material for a hybrid light-emitting diode, wherein said rigid material composition comprises a hydrophilic thermoplastic polymer having a glass transition temperature higher than 30°C and a luminescent protein immobilized therein.

In accordance with this aspect, the invention also refers to the use of the rigid material composition comprising a hydrophilic thermoplastic polymer having a glass transition temperature higher than 30°C and a luminescent protein immobilized therein, as a color down-converting cascade energy transfer encapsulation for a hybrid LED. Such a cascade energy transfer encapsulation typically comprises or consists of several layers (e.g., two, three, four, five, six, seven, eight, or more layers) of the rigid material containing a luminescent protein immobilized therein, wherein the luminescent proteins in each pair of neighbouring layers have complementary absorption and emission features. The bottom layers may, e.g., emit high energy photons upon excitation from the inorganic LED that are partially converted by the top layer into low-energy photons. The combination of the non-absorbed high-energy photons from the inorganic LED, the bottom down-converting layer, and the top down-converting layer preferably results in a white LED. The rigid material containing a luminescent protein immobilized therein can thus be used for fabricating white light-emitting diodes using an innovative cascade energy transfer encapsulating system, which circumvents the problems related to phase separation and exciton-loss in multicomponent single-layer down-converting encapsulation systems. The encapsulation system features a higher rendering color with advantageous stabilities under high luminance inputs and advantageous luminous efficiencies when the device is running under ambient conditions.

Furthermore, the light output of the device can be easily modified by the thickness of the rigid encapsulation material, thus covering the whole visible spectrum.

## Examples

Example 1. Preparation of fluorescent proteins.

The fluorescent proteins were prepared following a process consisting of two steps: Cloning of recombinant gene constructs and preparation of fluorescent proteins.

*Cloning of recombinant gene constructs*

To combine the different protein domains and to create the pQE-9 expression constructs, the overlap-PCR method was performed. Using gene specific oligonucleotides eGFP was fused to the SH2-domain (eGFP), mCherry was fused to the SH3-domain (mCherry) and mTagBFP was fused to the PABC-domain (mTagBFP). Fluorescent proteins and protein interaction domains were separated by glycine-serine linker sequences allowing proper folding of both protein domains. The extension of the proteins results in larger and more stable fusion proteins. After PCR and gel extraction the DNA fragments were ligated into the pQE-9 E. coli expression vector that contains an N-terminal 6xHis affinity tag, using T4 DNA ligase. The right orientation of the constructs and the N-terminal in frame fusion with the 6xHis tag were guaranteed using specific restriction enzymes. After the ligation, the recombinant plasmids were transformed into XL1 Blue E. coli cells and the correct sequence of the constructs was verified using Sanger Sequencing (GATC). For expression of the recombinant proteins pQE-9 plasmids, harboring the respective gene constructs, were transformed into E. coli M15 cells carrying the pREP4 repressor plasmid. Transformed E. coli cells were selected on plates containing ampicillin (pQE-9 expression vector, 200 $\mu$g/ml) and kanamycin (pREP4 repressor plasmid, 25 $\mu$g/ml).

*Preparation of fluorescent proteins*

E. coli strain M15 [pREP4] harboring the appropriate plasmids (pQE-9 expression constructs all containing a N-terminal 6xHis-tag coming from the pQE-9 expression vector, Qiagen) were grown at 28°C in Lysogeny Broth (LB) medium (Bertani G, J Bacteriol. 1951, 62(3), 293-300) containing ampicillin (200 $\mu$g/ml) and kanamycin (25 $\mu$g/ml) antibiotics to an optical density of approximately 0.5 at 600 nm. Recombinant protein expression was induced with 1 mM isopropyl $\beta$-D-1-thiogalactopyranoside at 28°C. After 4 h of induction at 28°C, cells were harvested and frozen at -20°C. Frozen bacteria cells were thawed and lysed by lysozyme treatment and sonication. Recombinant proteins were affinity purified by Ni-NTA affinity chromatography under native conditions, according to instructions of the manufacturer (QIAGEN). The concentration of the resulting purified proteins was determined by measuring the absorption at 280 nm using a NanoDrop Spectrophotometer ND-1000 (Peqlab). The purified proteins were dissolved/stored in elution buffer (50 mM NaH2PO4, pH 8.0; 300 mM NaCl; 250 mM imidazole) until further use.

Example 2. Preparation of a rigid color down-converting encapsulation system onto a LED chip

The first step consisted in the preparation of the protein-based gel-like material. Said protein-based gel was obtained by first dissolving 360 mg of a poly(vinyl alcohol) (PVA), commercially available under the name Mowiol 10-98 (PVA having a deacetylation degree of 98% and a viscosity of 10.000 cp) and having Mn of 61000, in 6 mL of water under high temperature and stirring conditions. Subsequently, an aqueous solution containing 1 mg of the fluorescent protein obtained according to example 1 was added to the PVA aqueous solution.

The resulting mixture was stirred for 5 minutes at 600 rpm. The mixture was left at room temperature overnight thus forming a gel-like material.

Once the protein-based gel-like material was obtained, this was deposited onto a light-emitting chip wetting completely its surface. In particular, a commercial blue emitting LED chip (purchased by Luxeon) with an electroluminescent spectrum at 450 nm was used in this example.

The resulting coated LED was transferred to a vacuum chamber under 1 mbar for a few hours. This drying step led to the formation of a rigid encapsulation coating onto the LED chip. This procedure was repeated to enhance the light down-conversion efficiency of the Bio-LED with respect to the thickness of the rubber-like coating, which was 1-2 mm.

The same procedure was followed but using other PVAs, namely those commercially available under the names Mowiol 28-99 (acetylation degree: 99% and viscosity: 28.000 cp), Mowiol 56-98 (acetylation degree: 98% and viscosity: 56.000 cp) and Mowiol 18-88 (acetylation degree: 88% and viscosity: 18.000 cp).

As well, the use of hydroalcoholic solutions was also investigated. The FP-coating with Mowiol 18-88 and fluorescent protein was prepared using a mixture of water/2-isopropanol in a 1:1 volume ratio. As shown in Figure 5, the stability of the FP-coating is not affected, while the heat generation is effectively reduced.

Finally, the same procedure was followed but using a poly(vinyl alcohol-co-ethylene) in a mixture of water/2-isopropanol in a 1:1 volume ratio. Figure 6 displays an excellent stability due to the reduction of the heat generation under device operation conditions.

Comparative example 1. Preparation of a rubber-like color down-converting encapsulation system onto a LED chip

For comparative purposes, a color down-converting encapsulation system was prepared according to that described in WO2016/203028. Thus, a protein-based gel was also prepared by mixing 120 mg of trimethylolpropane ethoxylate (TMPE) having a Mn of 450 Da with 20 mg of linear poly(ethylene oxide) having Mn of $5 \times 10^6$ Da, and adding to the resulting mixture 200 $\mu$L of an aqueous solution containing 0.15 mg of the fluorescent protein as obtained in example 1 while stirring vigorously at 1500 rpm overnight. The mixture was left at room temperature thus forming a gel-like material.

Once the protein-based gel-like material was obtained, this was deposited onto a light-emitting chip wetting completely its surface. In particular, a commercial blue emitting LED chip (purchased by Luxeon) with an electroluminescent spectrum at 450 nm was used in this example.

The resulting coated LED was transferred to a vacuum chamber under 1 mbar for a few hours. This drying step led to the formation of a rubber-like encapsulation coating onto the LED chip. This procedure was repeated to enhance the light down-conversion efficiency of the Bio-LED with respect to the thickness of the coating which was 1-2 mm.

Example 3. Characterization of the Bio-LEDs

The Bio-LEDs prepared according to example 2 and comparative example were characterized by using a Keithley 2400 as a current source applying 200 mA, while the luminous efficiency and changes of the electroluminescence spectrum were monitored by using Avantes spectrophotometer (Avaspec-ULS2048L-USB2) in conjunction with a sphere Avasphere 30-Irrad.

Figures 2a and 2b show the device performance of a Bio-LED having a rubber-like color down-converting encapsulation system without fluorescent protein and with fluorescent protein, respectively. More particularly, said figures plot the emission intensity of the fluorescent protein and the temperature of the coating upon driving at currents of 200 mA. As can be observed, when the fluorescent protein is present, the encapsulation system reaches a higher temperature. This negatively affects the viability of the fluorescent proteins, leading to a significant decrease in its luminescence over time. In fact, the decrease of temperature over time is the result of the loss of luminescence.

On the contrary, and as derivable from Figure 3, when a rigid color down-converting encapsulation system based on a hydrophilic polymer is used according to the invention, a lower temperature (less than 50 °C) is observed under the device working conditions, which is maintained over time. In fact, the loss of luminescence is almost five orders of magnitude lower when compared to the Bio-LED having the rubber-like coating

Furthermore, and as derivable from Figures 3, 4 and 5, the stability of a Bio-LED having the rigid color down-converting encapsulation system according to the invention is enhanced upon further reducing the temperature of the coating below 40 °C, reaching a stability of 250 h (regardless of the type of solvent) compared to the reference Bio-LED with a rubber-like polymer coating (5 min). Likewise, Figure 6 displays a strong enhancement of the device stability using FP-coating based on PVA co-polymer, showing the same trend with respect to reduction of the heat generation and enhancement of the FP emission stability in Bio-LEDs operating at high applied currents.

These results point out that the sole presence of a hydrophilic thermoplastic polymer having a Tg higher than 30°C provides high thermal- and photo-stabilities to the color down-converting encapsulation system, thus reducing drastically the degradability of fluorescent proteins and making Bio-LEDs surprisingly more stable over time.

## Claims

1. A hybrid light emitting-diode comprising a light emitting diode and a rigid color down-converting encapsulation material, said rigid color down-converting encapsulation material comprises at least one layer of a rigid material composition, wherein said rigid material composition comprises at least one rigid hydrophilic thermoplastic polymer having a glass transition temperature higher than 30°C, and a luminescent protein immobilized therein.

2. The hybrid light emitting-diode according to claim 1, wherein the rigid material composition comprises one rigid hydrophilic thermoplastic polymer having a glass transition temperature higher than 30°C and a luminescent protein immobilized therein.

3. A hybrid light emitting-diode comprising a light emitting diode and a rigid color down-converting encapsulation material, said rigid color down-converting encapsulation material comprises at least one layer of a rigid material composition, wherein said rigid material composition consists of a rigid hydrophilic thermoplastic polymer having a glass transition temperature higher than 30°C and a luminescent protein immobilized therein.

4. The hybrid light emitting-diode according to any one of claims 1 to 3, wherein the rigid material composition has a transparency higher than 80% in the visible spectra.

5. The hybrid light emitting-diode according to any of claims 1 to 4, wherein the rigid material composition has a refractive index equal or higher than 1.

6. The hybrid light emitting-diode according to any of claims 1 to 5, wherein the hydrophilic polymer is poly(vinyl alcohol), a derivative thereof or its copolymers.

7. The hybrid light emitting-diode according to claim 6, wherein the hydrophilic polymer is poly(vinyl alcohol).

8. The hybrid light emitting-diode according to any of claims 1 to 7, wherein the luminescent protein is a fluorescent protein.

9. A process for preparing the hybrid light emitting-diode as defined in claims 1 to 8, said process comprises:

   a) preparing an aqueous solution or a hydroalcoholic solution of a hydrophilic polymer having a glass transition temperature higher than 30°C;
   b) preparing an aqueous solution of a luminescent protein;
   c) mixing the solutions prepared in steps a) and b) to obtain a hydrogel;
   d) depositing the hydrogel obtained in step c) onto a light emitting diode;
   e) drying the hydrogel to obtain a rigid material composition containing the luminescent protein immobilized therein.

10. The process according to claim 8 wherein the hydrophilic polymer is poly(vinyl alcohol), a derivative thereof or a copolymer thereof.

11. The process according to claims 9 or 10, wherein the hydrogel is dried under vacuum conditions.

12. A hybrid light emitting-diode obtainable by the process as defined in claims 9 to 11.

13. Use of a rigid material composition as an environmentally friendly color down-converting encapsulation material for a hybrid light-emitting diode, wherein said rigid material composition comprises at least one hydrophilic thermoplastic polymer having a glass transition temperature higher than 30°C and a luminescent protein immobilized therein.

14. Use according to claim 13, wherein the hydrophilic polymer is poly(vinyl alcohol), a derivative thereof or a copolymer thereof.

15. Use according to claims 13 or 14, wherein the luminescent protein immobilized in the rigid material composition is a fluorescent protein.

Figure 1

Figure 2

(a)

(b)

Figure 3

(a)

(b)

Figure 4

(a)                    (b)

Figure 5

(a)                    (b)

Figure 6

Europäisches
Patentamt
European
Patent Office
Office européen
des brevets

**EUROPEAN SEARCH REPORT**

Application Number

EP 19 38 2428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/203028 A1 (FRIEDRICH-ALEXANDER-UNIVERSITÄT ERLANGEN-NÜRNBERG [DE]) 22 December 2016 (2016-12-22) * the whole document * | 1-15 | INV. H01L33/50 C07K17/04 |
| A | EP 2 546 320 A1 (KONINKL PHILIPS ELECTRONICS NV [NL]) 16 January 2013 (2013-01-16) * claims 1-15 * | 1-15 | |
| A | MICHAEL D. WEBER ET AL: "Bioinspired Hybrid White Light-Emitting Diodes", ADVANCED MATERIALS, 13 August 2015 (2015-08-13), pages n/a-n/a, XP055216230, ISSN: 0935-9648, DOI: 10.1002/adma.201502349 * the whole document * | 1-15 | |
| E | EP 3 495 393 A1 (FUNDACION IMDEA MAT [ES]) 12 June 2019 (2019-06-12) * the whole document * | 1-15 | TECHNICAL FIELDS SEARCHED (IPC) H01L C07K |

The present search report has been drawn up for all claims

| Place of search | Date of completion of the search | Examiner |
|---|---|---|
| The Hague | 12 November 2019 | But, Gabriela-Ileana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 38 2428

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2019

| Patent document cited in search report | | Publication date | Patent family member(s) | | Publication date |
|---|---|---|---|---|---|
| WO 2016203028 | A1 | 22-12-2016 | CN | 108028302 A | 11-05-2018 |
| | | | EP | 3311424 A1 | 25-04-2018 |
| | | | JP | 2018521125 A | 02-08-2018 |
| | | | KR | 20180032568 A | 30-03-2018 |
| | | | US | 2018171032 A1 | 21-06-2018 |
| | | | WO | 2016203028 A1 | 22-12-2016 |
| EP 2546320 | A1 | 16-01-2013 | CN | 103649269 A | 19-03-2014 |
| | | | EP | 2546320 A1 | 16-01-2013 |
| | | | EP | 2732006 A1 | 21-05-2014 |
| | | | JP | 6157462 B2 | 05-07-2017 |
| | | | JP | 2014531739 A | 27-11-2014 |
| | | | TW | 201309977 A | 01-03-2013 |
| | | | US | 2014125222 A1 | 08-05-2014 |
| | | | WO | 2013008125 A1 | 17-01-2013 |
| EP 3495393 | A1 | 12-06-2019 | EP | 3495393 A1 | 12-06-2019 |
| | | | WO | 2019115525 A1 | 20-06-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017039406 A

- WO 2016203028 A

**Non-patent literature cited in the description**

- **PARK JK et al.** *Appl Phys Lett.,* 2004, vol. 84, 1647
- **JANG HS et al.** *Appl Phys Lett.,* 2007, vol. 90, 041906
- **XIE R-J et al.** Nitride Phosphors and Solid-State Lighting. CRC Press, 2011
- **TOLHURST TM et al.** *Adv Opt Mater.,* 2015, vol. 3, 546
- **ZHANG R et al.** *Laser Photon Rev.,* 2014, vol. 8, 158
- *J. Am. Ceram. Soc.,* 2014, vol. 97, 1327-1352
- 1. *J. Sol. State Light,* 2014, 11
- *Environ. Health Perspect,* 2014, vol. 122, 269
- *Curr. Pharm. Des.,* 2015, vol. 21, 3428
- *Neuroscience,* 2016, vol. 339, 296
- In Innovative Materials: Engineering and Applications II. Key Engineering Materials. Trans Tech Publications, 2017, vol. 730, 112
- *Lighting Research & Technology,* 2017, https://doi.org/10.1177/1477153517708597
- **HELIOTIS G et al.** *Adv Mater,* 2006, vol. 18, 334
- **GU E. et al.** *Appl Phys Lett.,* 2007, vol. 90, 031116
- **HUYAL IO et al.** *J Mater Chem.,* 2008, vol. 18, 3568
- **KIM O et al.** *ACS Nano,* 2010, vol. 4, 3397
- **STUPCA M et al.** *J Appl Phys.,* 2012, vol. 112, 074313
- **BAN D et al.** *Phys Status Solidi Curr Top Solid State Phys.,* 2012, vol. 9, 2594
- **JANG E-P et al.** *Nanotechnology,* 2013, vol. 24, 045607
- **FINDLAY NJ et al.** *J Mater Chem C,* 2013, vol. 1, 2249
- **LAI C-F et al.** *Opt Lett,* 2013, vol. 38, 4082
- **CHEN J et al.** *J Mater Sci.,* 2014, vol. 49, 7391
- **KIM J-Y.** *Opt Commun.,* 2014, vol. 321, 86
- **SHEN P-C et al.** *Sci Rep.,* 2014, vol. 4, 5307
- **FINDLAY NJ et al.** *Adv Mater,* 2014, vol. 26, 7290
- *Adv. Mater,* 2006, vol. 18, 334
- *J. Mater. Chem.,* 2008, vol. 18, 3568
- *ACS Nano,* 2010, vol. 4, 3397

- *Nanotechnology,* 2013, vol. 24, 045607
- *Sci. Rep.,* 2014, vol. 4, 5307
- *Adv. Mater.,* 2014, vol. 26, 7290
- *Nanoscale,* 2015, vol. 7, 12045
- *Nat. Commun.,* 2013, vol. 4, 2717
- *Adv. Funct. Mater.,* 2015, vol. 25, 4796
- *J. Mater. Chem. C,* 2015, vol. 3, 3536
- *ACS Appl. Mater. Interfaces,* 2015, vol. 7, 15830
- *Mater. Horiz.,* 2016, vol. 3, 340
- *Adv. Mater.,* 2015, vol. 27, 5493
- *Adv. Funct. Mater,* 2017, vol. 27, 1601792
- **HUI, K.N.** *Nanotechnology,* 2008, vol. 19, 355203
- *Fen Bilim. Enstitüsü Derg.,* 2016, vol. 20, 490
- *Nanotechnology,* 2016, vol. 27, 45LT01
- *ACS Omega,* 2018, vol. 3, 15829
- *Nanotechnology,* 2008, vol. 19, 355203
- *Int. J. Biol. Macromol,* 2017, vol. 104, 43
- *J. Appl. Polym. Sci.,* 2017, vol. 134, 45324
- *Int. J. Biol. Macromol.,* 2017, vol. 96, 340
- *Carbohydr. Polym.,* 2015, vol. 127, 64
- *Int. J. Polym. Mater.,* 2010, vol. 56 (10), 786
- *PNAS,* 2005, vol. 102 (27), 9511
- *Proc SPIE Int. Soc. Opt. Eng.,* 2015, vol. 9554, 95540G-1
- *Chem. Phys. Lett.,* 2008, vol. 457, 408
- *Proc. SPIE,* 2006, vol. 6098
- *Otolaryngol. Head Neck Surg.,* 2016, vol. 154 (6), 1106
- *Biofouling,* 2000, vol. 15 (1-3), 109
- *Curr. Protoc. Cell Biol.,* 2009, vol. 10, 15
- *Langmuir,* 2015, vol. 21 (23), 10253
- *J. Biomater. Sci. Polym. Ed.,* 2012, vol. 15 (9), 1181
- *Front. Optoelectron.,* 2012, vol. 5 (2), 147
- *Chem. Phys. B,* 2010, vol. 19 (11), 118103
- *Adv. Funct. Mater.,* 2017, vol. 27, 1601792
- **REDDY CHICHILI VP et al.** *Protein Scí.,* 2013, vol. 22 (2), 153-67

- **BERTANI G.** *J Bacteriol.,* 1951, vol. 62 (3), 293-300